(19) Europäisches Patentamt
European Patent Office
Office européen des brevets

(11) Veröffentlichungsnummer : **0 501 592 A1**

(12) # EUROPÄISCHE PATENTANMELDUNG

(21) Anmeldenummer : **92200701.8**

(51) Int. Cl.[5] : **C07D 409/14,** A61K 31/41

(22) Anmeldetag : **02.03.92**

(30) Priorität : **01.03.91 DE 4107038**

(43) Veröffentlichungstag der Anmeldung :
**02.09.92 Patentblatt 92/36**

(84) Benannte Vertragsstaaten :
**PT**

(71) Anmelder : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Bohlmann, Rolf, Dr.**
**Kühler Weg 6 a**
**W-1000 Berlin 19 (DE)**
Erfinder : **Strehlke, Peter, Dr.**
**Droysenstrasse 10 a**
**W-1000 Berlin 12 (DE)**
Erfinder : **Muhn-Seipoldy, Hans-Peter, Dr.**
**Am Waldidyll 23**
**W-1000 Berlin 28 (DE)**
Erfinder : **Nishino, Yukishige, Dr.**
**Lanzendorfer Weg 14**
**W-1000 Berlin 22 (DE)**
Erfinder : **Schneider, Martin, Dr.**
**Schuchseestrasse 6 a**
**W-1000 Berlin 28 (DE)**

(54) **Triheteroarylmethane, Verfahren zu deren Herstellung, pharmazeutische Präparate, die diese enthalten sowie ihre Verwendung zur Herstellung von Arzneimitteln.**

(57) Es werden Triheteroarylmethane der allgemeinen Formel I

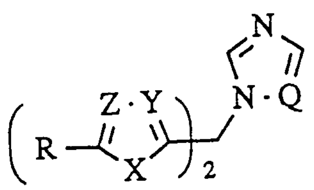
(I)

beschrieben, worin

R eine Cyano- oder $CF_3$-Gruppe, ein Fluor-, Chlor- oder Bromatom, einen Alkoxyiminorest -CH=N-OR′ oder einen Säureamidrest -C(O)NR′R″, wobei R′ und R″ unabhängig voneinander je für einen gerad- oder verzweigtkettigen Kohlenwasserstoffrest mit 1 bis 6 Kohlenstoffatomen stehen,

Q eine Methingruppe =CH- oder ein Stickstoffatom,

X ein Schwefel- oder Sauerstoffatom sowie

Y und Z unabhängig je eine Methingruppe =CH- oder ein Stickstoffatom

bedeuten,

sowie Verfahren zu deren Herstellung.

Die neuen Verbindungen sind Inhibitoren der Estrogenbiosynthese (Aromatasehemmer) und zur Herstellung von Arzneimitteln geeignet. Sie können zur Behandlung von Krankheiten dienen, die durch Estrogene bedingt oder von Estrogenen abhängig sind.

EP 0 501 592 A1

Die vorliegende Erfindung betrifft Triheteroarylmethane der allgemeinen Formel I

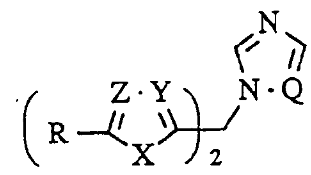

(I)

worin

R eine Cyano- oder $CF_3$-Gruppe, ein Fluor-, Chlor- oder Bromatom, einen Alkoxyiminorest -CH=N-OR′ oder einen Säureamidrest -C(O)NR′R″, wobei R′ und R″ unabhängig voneinander je für einen gerad- oder verzweigtkettigen Kohlenwasserstoffrest mit 1 bis 6 Kohlenstoffatomen stehen,

Q eine Methingruppe =CH- oder ein Stickstoffatom,

X ein Schwefel- oder Sauerstoffatom sowie

Y und Z unabhängig je eine Methingruppe =CH- oder ein Stickstoffatom bedeuten.

Vorzugsweise steht R für eine Cyanogruppe oder ein Chlor- oder Bromatom.

Bei R′und R″ ist in erster Linie an geradkettige, gesättigte $C_1$-bis $C_6$- Alkylreste gedacht. Für Y und Z ist je eine Methingruppe bevorzugt. X bedeutet, wenn Y und Z je eine Methingruppe sind vorzugsweise ein Schwefelatom.

Die nachstehenden Verbindungen sind erfindungsgemäß insbesondere bevorzugt:

1-Imidazolyl-di(5-brom-2-thienyl)-methan

1-Imidazolyl-di(5-chlor-2-thienyl)-methan

5-[(5-Cyan-2-thienyl)-(1-imidazolyl)-methyl]-thiophen-2-carbonitril

5-[(5-Cyan-2-thienyl)-(1,2,4-triazol-1-yl)-methyl]- thiophen-2-carbonitril

Ein wichtiges Merkmal der erfindergemäßen Triheteroarylmethane der allgemeinen Formel I ist es, daß sie zwei identische Reste R- als Substituenten am zentralen Kohlenstoffatom aufweisen.

Durch das somit achirale Kohlenstoffatom werden Probleme, wie sie sonst bei Racematen von chiralen Verbindungen anfallen, bei der Herstellung und Anwendung der erfindungsgemäßen Verbindungen vermieden.

Die Verbindungen der allgemeinen Formel I sind Inhibitoren der Estrogenbiosynthese (Aromatasehemmer). Sie sind damit zur Behandlung von Krankheiten geeignet, die durch Estrogene bedingt oder von Estrogenen abhängig sind. So sind sie geeignet zur Behandlung von östrogeninduzierten oder -stimmulierten Tumoren, wie zum Beispiel Mammakarzinom oder Prostatahyperplasie (The Lancet, **1984**, 1237-1239, J. Med. Chem. 33, **1990**, 2933).

Die erfingdungsgemäßen Verbindungen sind auch wertvoll zur Beeinflussung der Fertilität. So kann eine männliche Infertilität, die aus erhöhten Estrogenspiegeln resultiert, mit den neuen Wirkstoffen behoben werden. Ferner können die Verbindungen bei Frauen im fortpflanzungsfähigem Alter als Antifertilitätsmittel verwendet werden, um Ovolationen durch Estrogenentzug zu hemmen.

Wahrscheinlich sind Aromatasehemmer auch zur Behandlung des drohenden Herzinfarktes geeignet, da erhöhte Estrogenspiegel beim Mann einem Herzinfarkt vorangehen können (US-Patent 4,289,762).

Bekannte, eine aromatasehemmende Wirkung aufweisende Substanzen sind neben Steroiden auch nichtsteroidale Substanzen, beispielsweise die in den europäischen Patentanmeldungen, EP-A 0165777 bis 0165784 beschrieben diversen Stickstoffheterocyclen, die in J. Med. Chem. 1986, 29, Seiten 1362-1369 beschriebenen substituierten Glutarsäureimide, die in der europäischen Patentanmeldung EP 0165904 beschriebenen substituierten Imidazobenzole, die in der europäischen Patentanmeldung EP-A 0236940 beschriebenen substituierten heterocyclisch substituierten Toluolnitrile sowie die aus dem US-Patent US-A-4,728,465 hervorgehenden, einen gegebenenfalls substituierten Phenylring tragenden Imidazo- und 5,6,7,8-Tetrahydroimidazo[1,5a]pyridine, aus denen insbesondere das 5-(p-Cyanophenyl)-5,6,7,8-tetrahydroimidazo[1,5a]pyridin, Hydrochlorid als stark wirksamer Aromatasehemmer herausragt (Cancer Res. 48, S. 834-838, 1988).

Die Verbindungen der vorliegenden Anmeldung zeichnen sich gegenüber den bisher bekannten Verbindungen dadurch aus, daß sie das Enzymsystem der Aromatase stärker und zugleich selektiver hemmen. Die selektive Wirkung zeigt sich daran, daß andere Enzymsysteme in geringerem Umfang beeinträchtigt werden.

Die zu verabreichende Menge der Verbindungen schwankt innerhalb eines weiten Bereichs und kann jede wirksame Menge abdecken. In Abhängigkeit des zu behandelnden Zustands und der Art der Verabreichung kann die Menge der verabreichten Verbindungen 0.0001-10 mg/Kg Körpergewicht, vorzugsweise 0.001 - 1 mg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zuk-

ker, Sorbit, Gelantine, Gleitmittel, Kieselsäure, Talkum usw., enthalten. Die einzelnen Dosierungseinheiten für die orale Applikation können beispielsweise 0,05-50 mg des Wirkstoffes (Aromatasehemmers) enthalten.

Zur parenteralen Verabreichung können die Wirkstoffe in einem physiologisch verträglichen Verdünnungmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiergemisches verwendet. Als Beispiele für verwendete Öle sind zu nennen: Olivenöl, Erdnußöl, Baumwollöl, Sojabohnenöl, Rizinusöl und Sesamöl.

Die Verbindungen lassen sich auch in Form einer Depotinjektion oder eines Implantatpräparates anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silicone, wie zum Beispiel Siliconkautschuk. Die Wirkstoffe können außerdem zur perkutanen Aplikation zum Beispiel in Pflaster eingearbeitet werden.

Die Erfindung betrifft somit auch pharmazeutische Präparate und die Verwendung der Verbindungen zur Herstellung dieser Präparate zur Behandlung von östrogen-bedingten Krankheiten.

Die Tumor-Hemmwirkung von Imidazoldervaten beruht auf einer Inhibierung P-450 abhängiger Enzymsysteme (vgl. z.B. J.P. Van Wanne und P.A.J. Janssen, J. Med. Chem. 32, **1988**, 2231). Auch die Wirkung antifungaler Therapeutika aus der Reihe der Imidazol- Triazolderivate beruht auf einer Blockade P-450-abhängiger biochemischer Reaktionen (loc. cit.). Ferner ist aus der Patentliteratur bekannt, daß Azol-Derivate sowohl antifungale wie auch tumorhemmende Wirkung zugleich haben (vgl. hierzu EPA 0165777, Eli Lilly). Die erfindungsgemäßen Verbindungen sollten daher auch eine antifungale Wirkung gegen human-, tier- und pflanzenpathogene Keime aufweisen.

Die Erfindung betrifft außerdem ein Verfahren zur Herstellung der Verbindungen der allgemeinen Formel I.

Dabei wird entweder

a) eine Verbindung der allgemeinen Formel II

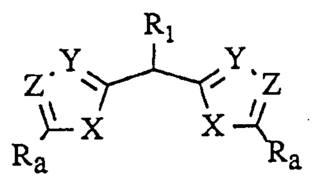

(II)

worin $R_a$ dieselbe Bedeutung wie R in Formel I hat oder für ein Wasserstoffatom steht und X, Y und Z die in Formel I angegebene Bedeutung haben sowie $R_1$ eine Hydroxyoder Abgangsgruppe bedeutet, mit einem den Rest

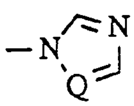

liefernden Reagens oder

b) eine Verbindung der allgemeinen Formel III

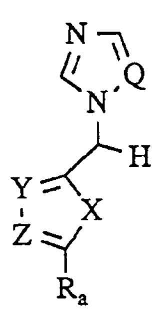

(III)

worin $R_a$, X, Y und Z dieselbe Bedeutung wie in der allgemeinen Formel II haben, unter Einwirkung einer Base und gegebenenfalls übergangsmetallkatalysiert mit einem den Rest

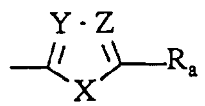

liefernden Reagens zu einer Verbindung der allgemeinen Formel Ia

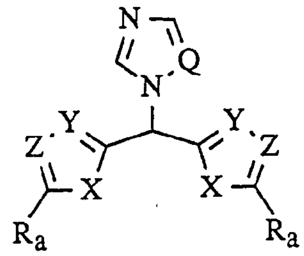 (Ia)

und anschließend wenn $R_a$ ein Wasserstoffatom bedeutet, $R_a$ nach gängigen Verfahren in einen Rest R oder wenn $R_a$ nicht für H steht, $R_a$ also bereits eine der unter R angegebenen Bedeutungen aufweist, gegebenenfalls in einen anderen unter R genannten Substituenten überführt.

Im allgemeinen ist die Abgangsgruppe $R_1$ in der Verbindung der allgemeinen Formel II eine Hydroxygruppe, weil sich diese Ausgangsverbindungen, wie noch zu beschreiben sein wird, gut herstellen lassen. Es ist jedoch zweckmäßig die Hydroxyabgangsgruppe vor der Umsetzung der Verbindung der allgemeinen Formel II mit einen den Rest

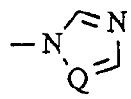

liefernden Reagens gegen eine bessere Abgangsgruppe auszutauschen.

Insbesondere ist hier die Überführung der Hydroxyfunktion in eine Chlorfunktion mit Thionylchlorid zu nennen. Als den Rest

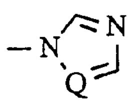

liefernde Reagenzien sind erfindungsgemäß 1,1-Carbonyl-di(1H-imidazol), 1,1-Carbonyl-ditriazol, 1,1-Sulfinyl-di(1H-imidazol), 1,1-Sulfinyl-ditriazol, N-Trialkylsilylimidazol, N-Trialkylsilyltriazol, aber auch Imidazol und 1,2,4-Triazol sowie Alkaliimidazolid bzw. Alkalitriazolid (Alkali = Lithium, Natrium) gut geeignet.

Die Umsetzung der Verbindung der allgemeinen Formel III mit einem den Rest

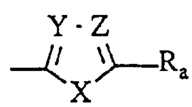

liefernden Reagens wird unter dem Einfluß einer starken Base durchgeführt, die in der Lage ist, ein Proton von zentralen Kohlenstoffatom aus der Verbindung der Formel III abzuspalten.

Zur Kupplung des aus der Verbindung der allgemeinen Formel III gebildeten Carbanions mit dem den Rest

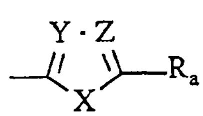

liefernden Reagens kann ein Übergangsmetallkatalysator wie zum Beispiel Tetrakisphenylphosphinpalladium

(0) verwendet werden. Als Quellen für den Rest

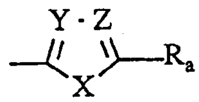

können die entsprechenden Brom- oder Fluorverbindungen (F)Br

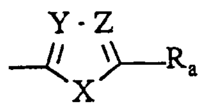

dienen; exemplarisch seien 5-Bromthiophen-2-carbonitril und 5-Fluorthiophen-2-carbonitril genannt.

Steht $R_a$ in der nunmehr erhaltenen Verbindung der allgemeinen Formel für Wasserstoff ist dieser nun noch gegen einen der unter R genannten Substituenten auszutauschen. Dazu wird zunächst chloriert oder bromiert und dann gewünschtenfalls die Halogenfunktion durch Umsetzung mit CuCN, $Pd^0$/Cyanid oder $Ni^0$/Cyanid in eine Cyanofunktion überführt. Steht $R^a$bzw. R für eine Cyanofunktion kann diese schließlich in an sich bekannter Weise in einer Alkoxyiminogruppe oder ein Säureamid umgewandelt werden.

Die nachstehenden Ausführungsbeispiele dienen der näheren Erläuterung der Erfindung. Aus ihnen ist auch die Herstellung von
Di(2-thienyl)-methanol (Beispiel 1a),
Di(5-chlor-2-thienyl)-methanol (Beispiel 2b),
1-(5-Brom-2-thienylmethyl)-imidazol (Beispiel 3a),
5-(1-Imidazolylmethyl)-thiophen-2-carbonitril (Beispiel 3b) sowie
5-(1,2,4-Triazol-1-ylmethyl)-thiophen-2-carbonitril (Beispiel 4a)
als Ausgangssubstanzen der allgemeinen Formeln II bzw. III zu entnehmen. Weitere Ausgangsverbindungen lassen sich in analoger Weise durch entsprechende Anpassung der Reaktionspartner erhalten.

**Beispiel 1**

1-Imidazolyl-di(5-brom-2-thienyl)-methan.

a) 1,1-Di(2-thienyl)-methanol.

12.6 g Thiophen in 100 ml Diethylether werden bei -10 °C mit 94 ml einer 1.6 molaren n-Butyllithiumlösung in Hexan langsam versetzt, 20 Minuten gerührt, auf -40 °C gekühlt und mit 10.58 ml Thiophenaldehyd in 20 ml Diethylether versetzt, 30 Minuten bei -40 °C gerührt, auf 25 °C erwärmt und 1 Stunde gerührt. Zur Aufarbeitung wird Eiswasser zugegeben, mit Diethylether extrahiert, mit gesättigter Kochsalzlösung gewaschen, über Natriumsulfat getrocknet und im Vakuum eingeengt. Man erhält 26 g rohes 1,1-Di(2-thienyl)-methanol.

b) 1-Imidazolyl-di(2-thienyl)-methan.

26 g rohes Di(2-thienyl)-methanol werden in 132 ml Tetrahydrofuran portionsweise mit 32.7 g 1,1′-Carbonyldi-1H-imidazol versetzt und 15 Minuten bei 25 °C gerührt.Dann wird langsam Wasser zugegeben, zwei mal mit Essigester extrahiert, mit Wasser gewaschen, über Natriumsulfat getrocknet und im Vakuum eingeengt. Nach der Chromatographie an Kieselgel erhält man 15.4 g reines 1-Imidazolyl-di(2-thienyl)-methan als leicht bräunliche Kristalle vom Schmelzpunkt 68-69 °C.

c) 1-Imidazolyl-di(5-brom-2-thienyl)-methan.

20 g 1-Imidazolyl-di(2-thienyl)-methan werden in 500 ml 48%iger wässriger Bromwasserstoffsäure bei -20 °C mit einer Lösung von 8 ml Brom in 24 ml 48%iger wässriger Bromwasserstoffsäure langsam versetzt und 24 Stunden bei 25 °C stehen gelassen. Dann wird mit 6N-Natronlauge alkalisiert, viermal mit Essigester extrahiert, mit gesättigter Kochsalzlösung neutralgewaschen, über Natriumsulfat getrocknet und im Vakuum eingeengt. Nach der Chromatographie an Kieselgel erhält man 15.8 g reines 1-Imidazolyl-di(5-brom-2-thienyl)-methan als leicht bräunliche Kristalle vom Schmelzpunkt 88-90 °C.

**Beispiel 2**

1-Imidazolyl-di(5-chlor-2-thienyl)-methan.

a) 5-Chlor-2-thiophencarbaldehyd.

3 g 2-Chlorthiophen in 25 ml Diethylether werden bei -78 °C Badtemperatur mit 15.8 ml einer 1.6 molaren *n*-Butyllithiumlösung in Hexan langsam versetzt, 1 Stunde gerührt, bei -50 °C mit 2.95 ml N-Formylmorpholin versetzt und 1 Stunde bei -50 °C gerührt. Zur Aufarbeitung wird das Reaktionsgemisch in eine Natriumhydrogencarbonatlösung gegeben, mit Diethylether extrahiert, mit gesättigter Kochsalzlösung gewaschen, über Natriumsulfat getrocknet und im Vakuum eingeengt. Man erhält 3.6 g rohen 5-Chlor-2-thiophencarbaldehyd als Öl.

b) Di(5-chlor-2-thienyl)-methanol.

2.7 g 2-Chlorthiophen in 30 ml Diethylether werden bei -78 °C Badtemperatur mit 14.3 ml einer 1.6 molaren n- Butyllithiumlösung in Hexan langsam versetzt, 1.5 Stunden gerührt, bei -50 °C mit 3 g 5-Chlor-2-thiophencarbaldehyd in 20 ml Diethylether versetzt und 15 Minuten bei -78 °C Badtemperatur gerührt. Zur Aufarbeitung werden 15 ml 2N- Salzsäure zugegeben, mit Wasser verdünnt, mit Diethylether extrahiert, mit gesättigter Kochsalzlösung neutralgewaschen, über Natriumsulfat getrocknet und im Vakuum zur Trockne eingeengt. Man erhält 5.6 g rohes Di(5-chlor-2-thienyl)-methanol.

c) 1-Imidazolyl-di(5-chlor-2-thienyl)-methan.

5.5 g rohes Di(5-chlor-2-thienyl)-methanol werden in 100 ml Tetrahydrofuran portionsweise mit 5.1 g 1.1′-Carbonylbis-1H- imidazol versetzt und 15 Minuten bei 25 °C gerührt. Dann wird langsam Wasser zugegeben, zweimal mit Essigester extrahiert, mit Wasser gewaschen, über Natriumsulfat getrocknet und im Vakuum eingeengt. Nach der Chromatographie an Kieselgel erhält man 4.4 g reines 1-Imidazolyl-di(5-chlor-2-thienyl)-methan als leicht bräunliche Kristalle vom Schmelzpunkt 75-76 °C.

**Beispiel 3**

5-[(5-Cyan-2-thienyl)-(1-imidazolyl)-methyl]-thiophen-2-carbonitril.

a) 1-(5-Brom-2-thienylmethyl)-imidazol.

20 g 5-Bromthiophen-2-carbaldehyd werden mit 4 g Natriumborhydrid in 100 ml 2-Propanol in üblicher Weise zu 20 g 5-Brom-2-thiophenmethanol reduziert. Dieses wird mit 30 ml Dichlormethan gelöst und mit 25 ml Thioylchlorid bei 25 °C 2 h gerührt. Nach Eindampfen im Vakuum und zweimaligem Nachdestillieren mit Toluol wird der Rückstand in 15 ml Dimethylformamid gelöst und zu einer aus 7.14 g Imidazol und 3.15 g Natriumhydrid-Ölsuspension (85%) in 30 ml Dimethylformamid bereiteten Lösung von Imidazolnatrium zugetropft. Nach 20 h bei 25 °C wird das Gemisch auf 2N-Salzsäure gegeben und mit Ether extrahiert. Die wässrige Phase wird mit Kaliumcarbonat alkalisiert und mit Essigester extrahiert, die organische Phase mit Wasser gewaschen, über Natriumsulfat getrocknet, am Vakuum eingeengt und am Kugelrohr destilliert. Man erhält 17,3 g 1-(5-Brom-2-thienylmethyl)-imidazol vom Siedepunkt 170 °C bei 0,03 mbar.

b) 5-(1-Imidazolylmethyl)-thiophen-2-carbonitril.

10 g 1-(5-Brom-2-thienylmethyl)-imidazol werden mit 10 g Kupfer(I)cyanid in 150 ml Dimethylformamid 20 h rücfluxiert. Dann wird das Gemisch auf Wasser gegeben, die Kristalle werden abgesaugt und mit 200 ml 25%igem Ammonikwasser eine Stunde bei Raumtemperatur gerührt. Dann wird mit Essigester extrahiert, mit Wasser gewaschen, über Natriumsulfat getrocknet, am Vakuum eingeengt und am Kugelrohr destilliert. Man erhält 6,2 g 5-(1-Imidazolylmethyl)-thiophen-2-carbonitril als orange Kristalle vom Schmelzpunkt 73-75 °C.

c) 5-[(5-Cyan-2-thienyl)-(1-imidazolyl)-methyl]-thiophen-2-carbonitril.

2.14 g 5-(1-Imidazolylmethyl)-thiophen-2-carbonitril werden in 50 ml Tetrahydrofuran bei -50 °C langsam mit 7.1 ml einer 2-molaren Lithiumdiisopropylamin versetzt und 30 Minuten bei -50 °C gerührt. Zu dieser Lösung werden bei -30 °C 517 mg Tetrakistriphenylphosphinpalladium(0) in 20 ml Tetrahydrofuran langsam zugetropft und 1 Stunde bei 25 °C gerührt. Nun werden 2.12 g 5-Bromthiophen-2-carbonitril zugegeben und bei 25 °C 2.5 Stunden gerührt. Zur Aufarbeitung wird die Reaktionslösung auf 1N-Salzsäure gegeben und mit Dichlormethan extrahiert. Die wässrige Phase wird mit 2N-Natronlauge auf pH 10 alkalisiert und mit Dichlormethan extrahiert, die organische Phase mit Wasser gewaschen, über Natriumsulfat getrocknet, im Vakuum zur Trockne eingeengt und an Kieselgel chromatographiert. Man erhält 1.5 g reines 5-[(5-Cyan-2-thienyl)-(1-imidazolylmethyl)-thiophen-2-carbonitril als Öl, das mit methanolischer Salpetersäure in das kristalline Nitrat vom Schmelzpunkt 78-81 °C überführt wird.

**Beispiel 4**

5-[(5-Cyan-2-thienyl)-(1,2,4-triazol-1-yl)-methyl]-thiophen-2-carbonitril.

a) 5-(1,2,4-Triazol-1-ylmethyl)-thiophen-2-carbonitril.

11 g 1,2,4-Triazol und 4.7 g Natriumhydrid in 100 ml Dimethylformamid werden mit 33 g 1-Brom-5-cyan-2-thienyl)methan unter Wasserbadkühlung versetzt und 20 Stunden bei 25 °C stehengelassen. Dann wird das Gemisch auf 2N-Salzsäure gegeben, zweimal mit Diethylether extrahiert, mit 2N-Salzsäure gewaschen, die vereinigten Wasserphasen mit Kaliumcarbonat alkalisiert, dreimal mit Essigester extrahiert, mit Wasser und gesättigter Kochsalzlösung gewaschen, über Natriumsulfat getrocknet, am Vakuum eingeengt und am Kugelrohr destilliert. Man erhält 6.2 g 5-(1,2,4-Triazol-1-ylmethyl)-thiophen-2-carbonitril als Öl mit dem Siedepunkt 205 °C bei 0.003 mbar.

b) 5-[(5-Cyan-2-thienyl)-(1,2,4-triazol-1-yl)-methyl]-thiophen-2-carbonitril.

2.14 g 5-(1,2,4-Triazol-1-ylmethyl)-thiophen-2-carbonitril werden in 50 ml Tetrahydrofuran bei -50 °C langsam mit 7.1 ml einer 2-molaren Lithiumdiisopropylaminlösung versetzt und 30 Minuten bei -50 °C gerührt. Zu dieser Lösung werden 517 mg Tetrakistriphenylphosphinpalladium(0) in 20 ml Tetrahydrofuran bei -30 °C langsam zugetropft und 1 Stunde bei 25 °C gerührt. Nun werden 2.12 g 5-Bromthiophen-2-carbonitril zugegeben und weitere 2.5 Stunden bei 25 °C gerührt. Zur Aufarbeitung wird die Reaktionslösung auf IN-Salzsäure gegeben und mit Dichlormethan extrahiert. Die wässrige Phase wird mit 2N-Natronlauge auf pH 10 alkalisiert und mit Dichlormethan extrahiert, die organische Phase mit Wasser gewaschen, über Natriumsulfat getrocknet, im Vakuum zur Trockne eingeengt und an Kieselgel chromatographiert. Man erhält 1.32 g reines 5-[(5-Cyan-2-thienyl)-(1,2,4-triazol-1-yl)-methyl]-thiophen-2-carbonitril als gelbliche Kristalle vom Schmelzpunkt 143- 144 °C.

**Patentansprüche**

**1)** Triheteroarylmethane der allgemeinen Formel I

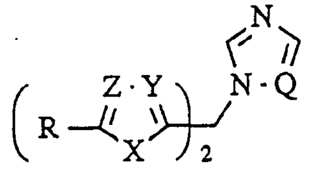 (I)

worin

R eine Cyano- oder $CF_3$-Gruppe, ein Fluor-, Chlor- oder Bromatom, einen Alkoxyiminorest -CH=N-OR′oder einen Säureamidrest -C(O)NR′R″, wobei R′ und R″ unabhängig voneinander je für einen gerad- oder verzweigtkettigen Kohlenwasserstoffrest mit 1 bis 6 Kohlenstoffatomen stehen,

Q eine Methingruppe =CH- oder ein Stickstoffatom,

X ein Schwefel- oder Sauerstoffatom sowie

Y und Z unabhängig je eine Methingruppe=CH- oder ein Stickstoffatom bedeuten.

**2)** Triheteroarylmethane nach Ausspruch 1, dadurch gekennzeichnet, daß R für eine Cyanogruppe, ein Chlor- oder Bromatom steht.

**3)** Triheteroarylmethane nach Ausspruch 1, dadurch gekennzeichnet,daß R′ und R″ geradkettige, gesättigte $C_1$-bis $C_6$-Alkylreste bedeuten.

**4)** Triheteroarylmethane nach Ausspruch 1, dadurch gekennzeichnet, daß Y und Z je eine Methingruppe bedeuten.

**5)** Triheteroarylmethane nach Ausspruch 4, dadurch gekennzeichent, daß X für ein Schwefelatom steht.

**6)** Triheteroarylmethane nach Ausspruch 1,

1-Imidazolyl-di(5-brom-2-thienyl)-methan

1-Imidazolyl-di(5-chlor-2-thienyl)-methan

5-[(5-Cyan-2-thienyl)-(1-Imidazolyl)-methyl]-thiophen-2-carbonitril

5-[(5-Cyan-2-thienyl)-(1,2,4-triazol-1-yl)-methyl]-thiophen-2-carbonitril

**7)** Verfahren zur Herstellung von Heteroarylmethanen der allgemeinen Formel I

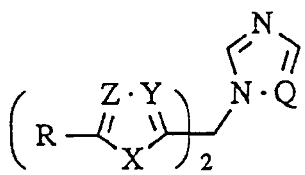

(I)

worin R, R′, R″, Q, X, Y und Z die in Ausspruch 1 angegebene Bedeutung haben, dadurch gekennzeichnet, daß, entweder

a) eine Verbindung der allgemeinen Formel II

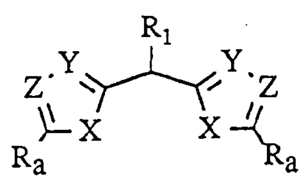

(II)

worin $R^a$ dieselbe Bedeutung wie R in Formel I hat oder für ein Wasserstoffatom steht und X, Y und Z die in Formel I angegebene Bedeutungen haben sowie $R^1$ eine Hydroxy- oder Abgangsgruppe bedeutet, mit einem den Rest-

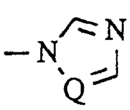

liefernden Reagens, oder

b) eine Verbindung der allgemeinen Formel III

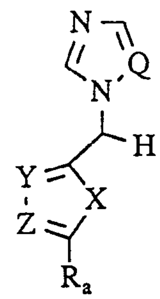

(III)

worin $R_a$, X, Y und Z dieselbe Bedeutung wie in der allgemeinen Formel II haben, unter Einwirkung einer Base und gegebenenfalls übergangsmetallkatalysiert mit einem den Rest

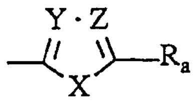

liefernden Reagens zu einer Verbindung der allgemeinen Formel Ia

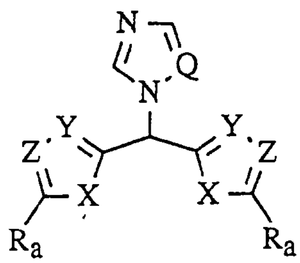

(Ia)

und anschließend wenn $R_a$ ein Wasserstoffatom bedeutet, $R_a$ nach gängigen Verfahren in einen Rest R oder wenn Ra nicht für H steht, Ra also bereits eine der unter R angegebenen Bedeutung aufweist, gegebenenfalls in einen anderen unter R genannten Substituenten überführt wird.

**8)** Verfahren nach Ausspruch 7, dadurch gekennzeichnet, daß die Hydroxyabgangsgruppe in der Verbindung der allgemeinen Formel II vor Umsetzung mit einem den Rest

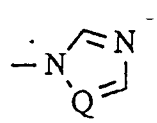

liefernden Reagens in eine bessere Abgangsgruppe überführt wird.

**9)** Verfahren nach Ausspruch 8, dadurch gekennzeichnet, daß die Hydroxyabgangsgruppe in ein Chloratom überführt wird.

**10)** Verfahren nach Ausspruch 7, dadurch gekennzeichnet, daß als

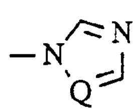

lieferndes Reagens 1,1-Carbonyl-di(1H-imidazol), 1,1-Carbonyl-di(triazol), 1,1-Sulfinyl-di(1H-imidazol), 1,1-Sulfinyl-ditriazol, N-Trialkylsilylimidazol, N-Trialkylsilyltriazol, aber auch Imidazol und 1,2,4-Triazol verwendet

wird.

**11)** Verfahren nach Ausspruch 7, dadurch gekennzeichnet, daß als

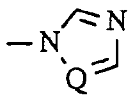

lieferndes Reagens Alkaliimidazolid oder Alkalitriazolid verwendet wird.

**12)** Verfahren nach Ausspruch 7, dadurch gekennzeichnet, daß zur Umsetzung der Verbindung der allgemeinen Formel III mit dem den Rest

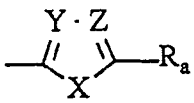

liefernden Reagens Lithiumdiisopropylamid als Base verwendet wird.

**13)** Verfahren nach Ausspruch 12, dadurch gekennzeichnet, daß als Übergangsmetallkatalystor Tetrakisphenylphosphinpalladium (0) zugesetzt wird.

**14)** Pharmazeutische Präperate, dadurch gekennzeichnet, daß sie mindestens ein Triheteroarylmethan gemäß der Ansprüche 1 bis 6 sowie einen pharmazenrischen Träger enthalten.

**15)** Verwendung der Triheteroarylmethane gemäß den Ausprüchen 1 bis 6 zur Herstellung von Arzneimitteln.

Europäisches Patentamt

# EUROPÄISCHER RECHERCHENBERICHT

Nummer der Anmeldung

EP 92 20 0701

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 337 928 (SCHERING AG)<br>* Ansprüche *<br>--- | 1,7-15 | C07D409/14<br>A61K31/41 |
| A | EP-A-0 411 735 (SCHERING AG)<br>* Ansprüche *<br>--- | 1,7-15 | |
| A | EP-A-0 371 564 (JANSSEN PHARMACEUTICA N.V.)<br>* Ansprüche *<br>----- | 1,7-15 | |
| | | | RECHERCHIERTE SACHGEBIETE (Int. Cl.5 ) |
| | | | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 12 MAI 1992 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)